Europäisches Patentamt

European Patent Office

Office européen des brevets

(11) Numéro de publication: **0 376 761 B1**

(12) **FASCICULE DE BREVET EUROPEEN**

(45) Date de publication de fascicule du brevet: **02.02.94** (51) Int. Cl.⁵: **A61F 13/15**

(21) Numéro de dépôt: **89402029.6**

(22) Date de dépôt: **17.07.89**

(54) **Article d'hygiène féminine à usage externe et son procédé de fabrication.**

(30) Priorité: **16.12.88 FR 8816614**

(43) Date de publication de la demande:
**04.07.90 Bulletin 90/27**

(45) Mention de la délivrance du brevet:
**02.02.94 Bulletin 94/05**

(84) Etats contractants désignés:
**AT BE CH DE ES FR GB GR IT LI LU NL SE**

(56) Documents cités:
**FR-A- 2 391 713**
**GB-A- 2 048 078**
**US-A- 3 093 546**
**US-A- 4 237 591**
**US-A- 4 547 195**

(73) Titulaire: **DIFINTER SA**
**Zone Industrielle**
**B.P. 40**
**F-34270 Saint Mathieu de Tréviers(FR)**

(72) Inventeur: **Tetu, Sylvie 68 Avenue de la Justice de Castelnau**
**Les Terrasses d'Occitanie - Bât.A**
**F-34090 Montpellier(FR)**

(74) Mandataire: **Burtin, Jean-François**
**Cabinet GEFIB**
**59, rue Edouard-Vaillant**
**F-92300 Levallois-Perret (FR)**

**Description**

La présente invention concerne des articles d'hygiène et notamment des articles d'hygiène féminine telles que définis dans le préambule de la revendication 1. Des telles articles sont connus par exemple du document US-A-4 547 195.

Elle a plus particulièrement des articles d'hygiène absorbants, présentant une activité bactériostatique et destinés à une protection périodique.

Elle a spécifiquement pour objet des articles hygiéniques assurant une protection périodique conformément à la revendication 1 et leur procédé de fabrication conformément à la revendication 8.

Univers longtemps tabou, le marché de l'hygiène féminine a beaucoup évolué ces dernières années.

Les changements dans les styles de vie : femmes plus actives ... travail, pratique régulière d'un sport, ont entraîné une évolution des consommatrices qui deviennent plus exigeantes quant à la qualité et à l'efficacité des produits.

Les grandes étapes dans l'évolution du marché des serviettes périodiques ont été :

- l'APPARITION DES "ULTRA MINCES" EN 1980 qui s'adressent à des femmes qui recherchent la discrétion, qui ont des flux normaux et qui ne lient pas les possibilités d'absorption à l'épaisseur.
- LA PRESENTATION SOUS SACHETS PLASTIQUES INDIVIDUELS EN 1982, ce qui facilite le transport et présente une connotation très "moderne".

On peut constater une tendance à la sophistication et une démarche de plus en plus cosmétique, tant dans les conditionnements qui deviennent plus accrocheurs, plus colorés, que dans le discours envers la consommatrice qui tend à se personnaliser.

HABITUDES ET ATTENTES DES CONSOMMATRICES :

17 millions de femmes utilisent des protections périodiques. 58 % préfèrent les protections externes et sont d'une manière générale fidèles à un type de produits.

A l'intérieur d'une même marque, plusieurs produits différents sont fréquemment utilisés.

Les principales qualités recherchées par les consommatrices sont :

- efficacité, discrétion, sécurité, confort et douceur

Les techniques de plus en plus perfectionnées font que les trois premières attentes sont généralement satisfaites. En ce qui concerne le confort, un fort élément psychologique entre en jeu. Beaucoup de femmes sont gênées par l'odeur éventuelle des règles qu'elles perçoivent d'une manière exacerbée alors qu'elles sont les seules à les sentir. Cette perception entraine un malaise, un manque de confiance en soi.

Pour pallier à cet inconvénient, deux solutions peuvent être envisagées :

. le parfumage des serviettes

. l'utilisation d'un produit déodorant.

Pour ces deux solutions, les réactions sont multiples. En effet, il y a une méfiance aussi bien envers des produits perçus comme très parfumés à l'ouverture du sachet que vers le terme déodorant qui sous entend un produit chimique actif et peut-être dangereux.

La solution à ce problème pourrait être l'utilisation d'un agent bactériostatique d'origine naturelle. La consommatrice serait alors rassurée quant à l'innocuité du produit. En outre, les produits naturels sont particulièrement en vogue dans l'univers de la cosmétique et les médias font largement écho sur leur efficacité. Mais, un agent bactériostatique à lui seul ne suffit pas pour résoudre le problème lié à l'odeur corporelle des femmes pendant la période menstruelle.

US-A-3 093 546 décrit effectivement l'emploi d'agents bactéricides comme les bisphenols halogénés incorporés dans la serviette périodique. Toutefois, comme ce type de composé est volatil, il est nécessaire d'ajouter un agent liant pour former une émulsion aqueuse que l'on applique ensuite sur la fibre du pansement ; on sèche ensuite les fibres et la couche bactéricide se trouve placée à la surface des fibres et non pas dispersée dans la masse du pansement. Autrement dit, la couche bactéricide est en contact direct avec les fluides cataméniaux.

La durée des règles est extrêmement variable d'une femme à l'autre, variant de 3 à 7 jours.

Par contre, pour une même personne, l'importance du flux menstruel est relativement constant. La quantité de sang et de débris tissulaires perdus au cours d'une menstruation donne un chiffre de 70 ml (60 à 200).

Il semble que l'âge de 35 ans sépare en 2 catégories. Les femmes qui ont des règles importantes étant les moins âgées.

L'écoulement menstruel présente une couleur rougeâtre caractéristique, dûe pour l'essentiel à la perte de sang et aux produits de dégradation des différentes muqueuses.

La composition des règles est extrêmement complexe. Différents auteurs s'accordent pour y reconnaître des produits des glandes de SKEEN et de BARTHOLIN, des glandes sudoripares et sébacées.

On rencontre également des produits d'exudation de la paroi vaginale, des cellules exfoliées, des glycoprotéines et différentes sécrétions provenant des oviductes et de la muqueuse utérine.

Naturellement, on retrouve dans les règles un grand nombre de micro-organismes, bactéries, levures et champignons. Les micro-organismes rencontrés sont d'une pathogénicité variable. Ils sont aérobies ou anaérobies suivant le cas, appartiennent au groupe des coques ou des bacilles et peuvent être gram positif ou gram négatif. C'est pourquoi, l'odeur que l'on peut attribuer à l'écoulement menstruel tient de différents facteurs dégradation des cellules exfoliées, produit du métabolisme bactérien, produits des glandes sudoripares et notamment l'acide lactique.

Certaines femmes sont sensibles au problème d'odeur lié au flux menstruel. Cette constatation doit tenir compte du contexte psychologique particulier lié à la survenue des règles. Une assise rationnelle de cette affirmation est cependant possible.

En effet, les odeurs proviennent de composés présents dans les sécrétions naturelles, qui peuvent produire ou fournir des substances qui seront utilisées comme substrats pour le métabolisme des bactéries.

D'une manière plus précise, on a identifié de l'acide lactique et de la 3-hydroxy-2-butanone ainsi qu'un certain nombre de dérivés aliphatiques à 2 et 5 atomes de carbone, connus pour leur odeur à très faible concentration.

Ainsi, toute substance susceptible de freiner le développement bactérien et fongique est capable de ralentir ou de supprimer la production de substances odorantes provenant essentiellement du métabolisme des micro-organismes.

L'odeur des substances indépendantes du métabolisme des micro-organismes sera piégée par un capteur d'odeur.

Il existe peu de travaux sur la population bactérienne des règles, mais on peut déduire une classification par extrapolation de la flore vaginale.

| CLASSIFICATION DES BACTERIES TROUVEES DANS LES SECRETIONS VAGINALES | | |
|---|---|---|
| GERMES | AEROBIE | ANAEROBIE |
| Gram+ cocci | Streptococcus<br>Staphylococcus<br>Micrococcus | Streptococcus<br>Peptostreptococcus<br>Peptococcus |
| Gram+ bacilli | Lactobacillus<br>Corynebacterium<br>Listeria<br>Mycobacterium | Lactobacillus<br>Propionibacterium<br>Clostridium<br>Eubacterium<br>Bifidobacterium<br>Actinomyces |
| Gram− cocci | Neisseria | Veillonella<br>Acidaminococcus |
| Gram− bacilli | Escherichia<br>Proteus<br>Klebsiella<br>Serratia<br>Citrobacter<br>Pseudomonas<br>Acinetobacter<br>Mycoplasma<br>Ureaplasma<br>Florabacterium<br>Alcaligenes | Bactéroïdes<br>Eusobacterium |

C'est pourquoi la présente invention se propose de résoudre ce problème d'odeur, d'efficacité d'absorption et de discrétion de l'article d'hygiène par une solution nouvelle.

La solution a été de réaliser des articles de protection conformément à la revendication 1, c.a.d. des serviettes périodiques ou les protège-slips contenant une substance bactériostatique et fongistatique pour arrêter le développement microbien et une substance, parfumée ou non, susceptible de capter les odeurs corporelles.

Le choix de la substance bactériostatique nécessite la vérification de l'absence de toxicité, de la parfaite tolérance cutanée et de l'absence du risque d'allergie. Il a donc été nécessaire de choisir parmi les nombreux agents bactériostatiques connus, un produit répondant à ces conditions essentielles. Les préparations à base d'acide usnique satisfont à ces trois conditions premières et c'est ce qui explique ce choix. Sous le vocable "préparations à base d'acide usnique", on entend soit des concentrés végétaux à base de mousse alpine (Usnea Barbata) titrés en acide usnique, soit l'acide Usnique lui-même, soit encore un sel de l'acide Usnique avec une base minérale ou organique physiologiquement compatible.

Un deuxième critère de choix est la solubilité du produit bactériostatique dans un solvant organique imbibant le tampon de fluff et non volatil. C'est la raison pour laquelle, on choisit de préférence un extrait non aqueux de mousses alpines titré en acide Usnique ou un sel de base organique de l'acide Usnique comme le sel de diéthylamine ou de triethylamino éthanol. Ces produits sont solubles dans les solvants organiques. Leur solution possède un pH voisin de la neutralité. Ils ne sont ni toxiques, ni irritants et sont parfaitement tolérés par la peau.

Le produit préféré est le concentré d'acide Usnique commercialisé par la firme SOPHIM (sous la dénomination Extrait actif 334 SOL) et COSMETOCHEM (sous la marque commerciale deposée DEO-USNATE) titré respectivement à 8 % en acide (+) usnique dans le dipropylène glycol et 7.5 % en acide (+) usnique dans le propylène glycol.

L'agent captant les odeurs est une substance chimique ou un mélange de substances chimiques de préférence d'origine naturelle, non toxiques, parfumées ou non parfumées, qui a pour propriété d'atténuer ou de faire disparaitre l'odeur propre des sécrétions. Il ne s'agit pas d'un déodorant, ni d'une substance anti-perspirante dont l'utilisation risquerait d'être mal tolérée ou même dangereuse car beaucoup de ces produits sont caustiques.

L'agent captant les odeurs a la faculté de posséder par lui-même, une odeur légère ou faible, à note fraîche tout en étant discrète mais qui se combine à l'odeur corporelle pour produire un mélange dont il ressort une impression de douceur et de bien-être. Le capteur d'odeur est donc bien plus un facteur d'ambiance agréable plutôt qu'un parfum. En outre, ce n'est pas une substance parfumante.

Le solvant organique capable d'imbiber le matériau en pâte à papier défibrée non volatil, est de préférence un glycol ou un dérivé de glycol. Ce sont des produits inertes chimiquement, très peu toxiques, bon solvants et dont le pouvoir d'impregnation est en permanence très satisfaisant.

On pourra citer, à cet égard, le propylène glycol, le dipropylène glycol, l'isosorbide, le tripropylène glycol, la glycérine, la diglycérine, le monopropylène glycol, le diéthylène glycol, le triéthylène glycol. Les conditions à remplir pour un tel agent sont un bon pouvoir solvant pour l'agent bactériostatique et fongistatique, et pour l'agent captant les odeurs, une viscosité relativement faible pour permettre la pulvérisation, un pouvoir d'imbibition élevé, une remanence importante et une absence de pouvoir irritant. Le propylène glycol et le dipropylène glycol sont à cet égard les agents préférés.

L'agent capteur d'odeur qui répond le mieux aux critères de fraîcheur et d'efficacité est la préparation dénommée Fragrance 10639 commercialisée par la firme LAUTIER SA. Toutefois, d'autres présentations qui satisfont également aux conditions posées ci-dessus pour le capteur d'odeur peuvent aussi bien être utilisées. De même, es capteurs d'odeur non parfumés peuvent également servir comme la chlorophylline ou d'autres dérivés incolores de la chlorophylle.

Les proportions relatives d'agent bactériostatique et d'agent capteur d'odeur peuvent varier dans de larges proportions et notamment entre 15 parties d'agent bactériostatique à 1 partie d'agent capteur d'odeur et 5 parties d'agent capteur d'odeur pour 1 partie d'agent bactériostatique. Ces rapports sont déterminés principalement en fontion du pouvoir bactériostatique et fongistatique du dérivé de l'acide Usnique employé et du pouvoir capteur, du capteur d'odeur employé.

L'imbibition du matériau en pâte à papier défibrée s'effectue par trempage, par pulvèrisation, par imprégnation du matériau ou par brumisation à l'aide d'une solution de l'agent bactériostatique et de l'agent capteur d'odeur dans le solvant approprié.

Le matériau ainsi imbibé est alors enveloppé dans une nappe d'ouate de cellulose. L'ensemble est replié en trois plis de sorte que la partie absorbante du matériau cellulosique est placé au centre de la masse absorbante.

La quantité de mélange d'imbibition par article de protection périodique est assurée par pulvérisation de la solution dans le propylène glycol pendant une période déterminée. La quantité d'agent bactériostatique étant de 1 à 10 mg en solution à 7.5%, le volume pulvérisé sera de l'ordre de 0,01 à 0,1 ml.

La quantité utile d'acide Usnique ou de ses dérivés varie de 1 à 10 mg par article de protection périodique. Elle est en général de 3 à 8 mg pour une serviette périodique normale, elle est de 2 à 6 mg pour une serviette périodique ultra mince, elle est de 1 à 6 mg pour un protègeslîp et elle est de 6 à 12 mg pour une serviette périodique de nuit.

La quantité de capteur d'odeur est proportionnelle au poids du produit par article de protection périodique varie de 0,1 mg à 1 mg par article. Il est de préférence pour la composition dénommée "Fragrance 10639" de 0,4 à 1 mg pour les serviettes périodiques normales, de 0,2 à 0,6 mg pour les serviettes périodiques ultra-minces, de 0,1 à 0,4 mg pour les protège-slips et de 0,7 à 1,2 mg pour les serviettes périodiques de nuit.

L'invention concerne également à titre de produits nouveaux les articles de protection périodique ainsi imbibés constitués par une nappe de pâte fluff que l'on recouvre d'une épaisseur d'ouate de cellulose, sur laquelle, on dépose par pulvérisation ou par imprégnation des micro-gouttelettes de la solution d'imbibition laminage et formation en trois plis superposés.

Le tampon ainsi imbibé est découpé à la forme puis recouvert d'une feuille de polyéthylène débordante aux extrémités, disposé à la surface externe du tampon et remontant sur les bords. L'ensemble est inséré dans un voile externe non tissé, replié et collé longitudinalement sur lequel sont appliqués des traits de Holt Melt permanent pour assurer l'adhésivité du produit. Cet adhésif permanent est ensuite protégé par un papier siliconé. Le produit fini est thermoscellé aux deux extrémités.

Dans le cas de la serviette périodique de nuit imbibée selon l'invention, la pâte fluff en trois plis est plus épaisse et la partie tampon est plus longue. La constitution de cet article de protection périodique est, par ailleurs, identique à celui des autres serviettes périodiques imbibées, selon l'invention.

L'invention sera illustrée par les exemples suivants, sans toutefois être limitée en aucune façon.

Solution d'acide Usnique

**DESCRIPTION :** La solution d'acide Usnique commercialisée sous le nom d'Extrait Actif 334 SOL est de couleur ambrée. Elle se présente sous la forme d'une solution de l'Extrait Actif 334 (poudre) dans du propylène glycol. La solution est dosée à 8 % d'extrait actif. Le principe actif est l'acide D-usnique, extrait de la mousse Usnéa barbata.

L'acide usnique présente un grand intérêt en cosmétique. Toutefois, son utilisation est rendue difficile par sa faible solubilité.

L'Extrait Actif 334 SOL est présenté en solution dans le propylène glycol afin de faciliter l'emploi du complexe poudre Extrait Actif 334 (acide usnique purifié et complexé).

L'Extrait Actif 334 SOL peut aussi exister en solution dans du dipropylène glycol.

**CARACTERISTIQUES PHYSICO-CHIMIQUES :**

| . Aspect | Solution de propylène glycol de couleur ambrée |
|---|---|
| . Odeur | Légère, caractéristique |
| . pH, 20 % dans l'eau | 7,9 |
| . Masse volumique, 20°C | 1,040 - 1,050 |
| . Indice de réfraction, n=8 | 1,4400 - 1,4600 |
| . Matière sèche, % | 8 ± 0,2 à 105°C |
| . Teneur en acide usnique | 5,2 ± 0,2 % |

. Solubilité :

Eau                              Soluble légèrement trouble

Ethanol                        Soluble

Myristate d'isopropyle      Soluble

| ELEMENTS TOXICOLOGIOUES | |
|---|---|
| . LD50, g/kg | > 5 |
| . Irritation cutanée primaire | non irritant |
| . Irritation oculaire primaire | non irritant |

L'activité antimicrobienne de l'acide usnique est décrite dans la littérature. Cette activité est surtout marquée sur les bactéries gram(+), levures et champignons, elle est moindre sur les bactéries gram(-).

| MICROORGANISMES | DOSE MINIMUM INHIBEE |
|---|---|
| G+ Staph. aureus | 1:160-320.000 |
| G+ Strept. haemolyticus | 160.000 |
| G+ Strept. pyogene | 250.000 |
| G+ Strept. faecalis | 100.000 |
| G+ Strapt. camosus | 60.000 |
| G+ Strept. viridans | 64.000 |
| G+ Dipht. pneumoniae I | 160.000 |
| G+ Mycobact. tuberculosis avium | 160.000 |
| G+ Mycobact. tuberculosis hom | 500.000 |
| G+ Mycobact. phlei | 200.000 |
| G+ Mycobact. smegmatis | 200.000 |
| G+ Sarcina. lutéa | 20.000 |
| G+ Sarcina. rosa | 64.000 |
| G+ Sarcina. citrea | 64.000 |
| G+ Bact. bifidus | 64.000 |
| G+ Bact. mycoides | 100.000 |
| G+ Bact. acidophilus | 100.000 |
| G+ Bact. anthracis | 100.000 |
| G+ Bact. mesentericus | 100.000 |
| G+ Enterococus | 100.000 |
| G+ N. gonorrhoeae | 40.000 |
| G+ H. pertussis | 20.000 |
| G- E. Coli | 1.000 |
| G- B. typhii | 1.000 |
| G- B. pyocynaeus | 1.000 |
| G- Proteus vulgaris | 1.000 |
| F Candida tropicalis | 5.000 |
| F Candida pulcherina | 15.000 |
| F Trichophyton interdigitalis | 10.000 |
| F Trichophyton violaceum | 10.000 |
| F Fomes ammosus | 10.000 |
| F Acorion gallinae | 10.000 |

| Fragrance 10639 | |
|---|---|
| EXPRESSION | nature, rosée, fleurie, musquée |
| EVOCATION | fraîcheur discrète d'églantine et de rose blanche |
| ASPECT | liquide jaune pâle |
| DENSITE | 1,000 à 1,025 |
| INDICE DE REFRACTION | 1,442 à 1,462 |
| SOLUBILITE | alcool 75°, propylène glycol, dipropylène glycol |
| POINT ECLAIR | + 112°C (AFNOR coupelle fermée) |
| STOCKAGE | à l'abri de la chaleur et de la lumière |

## EXEMPLE I

**Mode de réalisation d'une serviette hygiénique normale pliée, imbibée selon l'invention**

Caractéristiques du produit :

| . Poids total | 11 g ± 0,5 |
|---|---|
| . Longueur totale | 250 mm ± 10 |
| . Longueur du tampon | 205 mm ± 10 |
| . Largeur du tampon | 70 mm ± 5 |
| . Les deux extrémités sont thermoscellées et arrondies. | |

Composition du produit :

- Masse absorbante

La masse absorbante (1) est formée de pâte de cellulose à longues fibres non traitée (fluff) servant de drain. La pâte de cellulose ne comporte ni azurant optique ni d'agent mouillant pour augmenter son pouvoir absorbant.

La solution d'acide Usnique et de fragrance 10639 dans le propylène glycol est pulvérisée sur la pâte de cellulose à raison de 0,1 à 1 ml par article. Une nappe d'ouate de cellulose (2) également non traitée sans azurant optique et formée exclusivement de fibres naturelles est disposée autour de la masse absorbante (1) par laminage.

La nappe d'ouate de cellulose (2) est repliée en trois plis superposés. Une feuille de polyéthylène (3) est placée sur la surface du pli supérieur (4) débordant sur les extrémités de la nappe de cellulose. L'ensemble est enfermé dans un voile externe non tissé (7) sans azurant optique, débordant à chaque extrémité sur la nappe d'ouate de cellulose (2) et sensiblement de même longueur que la feuille de poly éthylène (3), on rabat le voile externe (7) vers le centre et colle les deux bords par trois traits de colle (8) disposés longitudinalement de façon que l'un des bords se superpose sur l'autre bord. Les extrémités du tissu non tissé (7) sont scellées par thermoscellage en arrondissant les bouts. On dépose à chaud un adhésif permanent (6) sur un protecteur siliconé (5) que l'on transfère sur la surface du voile externe. On coupe le produit fini afin d'obtenir des extrémités arrondies.

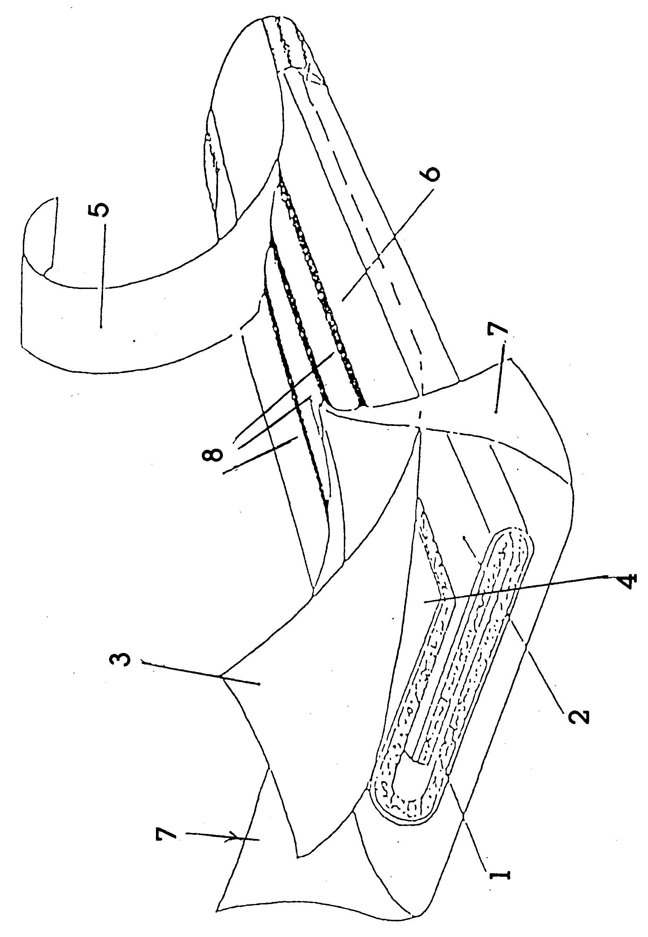

## Revendications

1. Articles d'hygiène féminine assurant une protection périodique et comportant un agent qui supprime les odeurs corporelles placé dans un matériau absorbant cellulosique replié, caractérisés par le fait qu'ils comportent une masse absorbante centrale à base de pâte à papier défibré imbibée par imprégnation, pulvérisation ou trempage d'une solution d'un agent bactériostatique et fongistatique et d'un agent captant les odeurs corporelles en les atténuant, dans un solvant hydrophile non volatil, adhérent aux fibres cellulosiques; que la pâte imbibée est enveloppée, par une nappe d'ouate de cellulose non traitée disposée tout autour de la masse absorbante et que celle-ci est repliée en trois plis superposés

pour former un tampon dont la partie absorbante et imprégnée est placée au centre de la masse cellulosique.

2. Article d'hygiène féminine selon la revendication 1° dans lequel l'agent bactériostatique et fongistatique est une préparation à base d'acide Usnique.

3. Article d'hygiène féminine selon les revendications 1° ou 2° dans lequel l'agent bactériostatique et fongistatique est un concentré végétal de mousse alpine titré en acide Usnique.

4. Article d'hygiène féminine selon l'une des revendications 1 ou 2° dans lequel l'agent bactériostatique et fongistatique est un sel d'acide Usnique avec une base minérale ou organique physiologiquement compatible.

5. Article d'hygiène féminine selon l'une des revendications 1 à 4° dans lequel l'agent captant les odeurs est un composé qui a pour propriété d'atténuer ou de faire disparaître l'odeur propre des secrétions sans être par lui-même un agent parfumant.

6. Article d'hygiène féminine selon l'une des revendications 1 à 5° dans lequel le solvant non volatil anhydre est un glycol choisi dans le groupe constitué par le propylène glycol, le dipropylène glycol, le diéthylène glycol, l'isosorbide, le tripropylène glycol, la glycérine, la diglycérine, le monopropylène glycol et le triethylène glycol.

7. Article d'hygiène féminine selon l'une des revendications 1 à 6° dans lequel les proportions d'agent bactériostatique et fongistatique à l'agent capteur d'odeur varie de 15 parties pour 1 partie à 1 partie pour 5 parties.

8. Procédé d'obtention d'un article d'hygiène féminine selon l'une des revendications 1 à 7° dans lequel sur une nappe de cellulose défibrée, on dépose par pulvérisation ou par imprégnation, une solution contenant un agent bactériostatique et fongistatique un agent captant les odeurs corporelles dans un solvant hydrophile anhydre, en quantité proportionnelle au poids de l'article, recouvre celle-ci d'une épaisseur d'ouate de cellulose, procède au laminage de ce matériau imprégné, puis au pliage en trois plis superposés, pour former un tampon que l'on recouvre d'une feuille de polyéthylène débordante aux extrémités, disposée à la surface externe du tampon et remontant sur les bords, insère cet ensemble dans un voile externe de tissu non tissé replié, de manière à e que les deux bords se superposent, que l'on colle longitudinalement puis recouvre sur la face externe par un protecteur siliconé collé sur les deux faces pour former un article d'hygiène féminine, scellé par thermosoudage aux deux extrémités.

## Claims

1. Goods for women's hygiene insuring a menstrual protection and incorporating an agent which suppresses the bodily odours located in a cellulosic absorbent material which is folded up, characterized in that they include a central absorbing mass of defibrated paper pulp, which is imbued through impregnation, spraying or soaking of a solution of a bacteriostatic and fungistatic agent together with an agent which seizes the bodily odours while weakening them, in a hydrophilic non-volatile solvent which adheres to the cellulosic fibers ; in that the imbued paste is wrapped with fluffed untreated wood pulp disposed all around the absorbing mass and in that the latter is pleated in three superimposed pleas to form a pad, the absorbing and impregnated part of which is located in the center of the cellulosic mass.

2. Good for women's hygiene according to claim 1° wherein the bacteriostatic and fungistatic agent is a composition based on Usnic acid.

3. Good for women's hygiene according to claims 1° or 2° wherein the bacteriostatic and fungistatic agent is a vegetal concentrate of Alpine Moss titrated into Usnic acid.

4. Good for women's hygiene according to anyone of claims 1° or 2° wherein the bacteriostatic and fungistatic agent is a base addition salt of Usnic acid with a physiologically compatible mineral or organic base.

5. Good for women's hygiene according to anyone of claims 1° to 4° wherein the agent which seizes the odours is a compound having the property to moderate or to let disappear the own odour of secretions whithout being by itself a perfuming agent.

6. Good for women's hygiene according to anyone of claims 1° to 5° wherein the non volatile anhydrous solvent is a glycol selected from the group consisting of propylene glycol, dipropylene glycol, diethylene glycol, isosorbide, tripropylene glycol, glycerol, diglycerol, monopropylene glycol and triethylene glycol.

7. Good for women's hygiene according to anyone of claims 1° to 6° wherein the ratio of bacteriostatic and fungistatic agent to the scavenger of odours, ranges from 15 parts for 1 part to 1 part for 5 parts.

8. A process for producing a good for women's hygiene according to anyone of claims 1° to 7° wherein, on a defibrated wood pulp sheet, a solution containing a bacteriostatic and fungistatic agent together with an agent which seizes the bodily odours in an anhydrous hydrophilic solvent, is laid down by spraying or by soaking, in an amount proportional to the weight of the good, covers the latter of a layer of paper wool, proceeds to the flattening of this impregnated good, then to the pleating in three superimposed pleas to form a pad which is covered with a sheet of polyethylene extending beyond the ends, located on the external face of the said pad and proceeding up on the borders, inserts this material in an external fleece of pleated non wowen cloth, in order that the two borders are superimposed, longitudinally sticked it, then covers it on the external face with a siliconized protective shield on which an adhering agent which sticks on both sides is applied to produce a good for women's hygiene which is sealed at both ends by thermosealing.

**Patentansprüche**

1. Waren für weibliche Hygiene die eine periodische Schutz gewahrleisteten und die ein Mittel das das körperliches Geruch unterbindet, einschliessen, das in einem zusammengeklappt, aufnehmende, auf Zellstoff basierte Material gesteckt wird, dadurch gekennzeichnet ist, dass sie eine zentrale, aufnehmende, aus entfasernten Zellstoff Masse enthalten die man mit einer Lösung eines bakteriostatischen und fungistatischen Mittels, sowie eines das das körperliches Geruch auf saugt in dem das abschwächt, Mittels in einen hydrophilen nicht flüchtigen, Lösungsmittel das auf der zellulosichen Fasern haftet durch Imprägnierung, Spruhen oder Eintauchen, durchtränkt, dass die durchgetränkte Masse durch einen Schicht von unbehandelte Zellstoff die um die aufnehmende Masse angeordnet wird umhüllt wird, und dass diese Masse in drei ubergedeckte Falten gefaltet ist um eine Propfen zu bilden, der aufnehmende und durchgetrankte Teil dessen in der Mitte der zellulosiche Masse gestellt wird.

2. Waren für weibliche Hygiene nach Anspruch 1° worin das bakteriostatischen und fungistatischen Mittel eine Zubereitung aus Usnin Säure ist.

3. Waren für weibliche Hygiene nach der Anspruch 1° und 2° worin das bakteriostatischen und fungistatischen Mittel ein planfzliche Konzentrat von alpischen Moos, das auf Usnin Säure getitriert wird, ist.

4. Waren für weibliche Hygiene nach einer der Ansprüche 1° oder 2°, worin das bakteriostatischen und fungistatischen Mittel, ein Salz von Usnin Säure mit einer physiologisch-verträglichen, anorganischen oder organischen Base ist.

5. Waren für weibliche Hygiene nach einer der Ansprüche 1° bis 4°, worin das Mittel das das Geruch aufsaugt, eine Verbindung ist, die die Eigenschaft besitzt das eigenes Geruch der Sekretionen abzuschwächen oder wegraümen ohne selbst ein düftendes Mittel zu sein.

6. Waren fur weibliche Hygiene nach einer der Ansprüche 1° bis 5° worin das wasserfreie, nicht flüchtige Lösungsmittel ein Glykol aus der Gruppe von Propylenglykol, Dipropylenglykol, Diethylenglykol, Isosorbid, Tripropylenglykol, Glyzerin, Diglyzerin, Monopropylenglykol und Triethylenglykol gewählt wird.

7. Waren für weibliche Hygiene nach einer der Ansprüche 1° bis 6° worin die Verhältnisse von des bakteriostatischen und fungistatischen Mittels zu dem das Geruch aufsaugenden Mittel, von 15 Teilen fur 1 Teil bis 1 Teil für 5 Teilen schwänkt.

8. Verfahren zur Herstellung einer Ware fur weiblichen Hygiene nach einer der Ansprüche 1° bis 7, worin man über einen Schicht von zellstoff (Fluff), eine Lösung eines bakteriostatischen und fungistatischen Mittels und eines Mittels das das körperlichen Geruch aufsaugt in einem hydrophilen wasserfreien Lösungsmittel, durch Sprühen oder Eintauchen, einer Menge proportional zum Gewicht der Ware, ablegt, man dieser Schnitt mit einem Schicht von Zellstoffwatte bedeckt, man das Walzen dieses eingetauchte Material, denn das Falten in drei uberdeckte Falten verfahrt, um ein Propfen zu bilden, der man mit einer in der Ende uberstehende Polyethylen Folie die auf die aüssere Oberfläche des Propfens angeordnet ist, bedeckt und die uber die Säume hinausgeht, man die Komplexen einen äussere Schleier von ungewebte Gewebe einführt das gefällt ist in der Weise dass die zwei Säume überbedeckt werden, man in der Langsrichtung verklebt, denn man auf die äussere Fläche mittels eines silikonischen Schutzmittel das auf beide Seite verklebt wird, bedeckt um eine Ware fur weiblichen Hygiene zu Schaffen die auf die beide Ende durch Thermosiegeln versiegelt wird.